# NEW EUROPEAN PATENT SPECIFICATION

(11) **EP 1 894 556 B2**
(45) Date of publication and mention of the opposition decision: **10.08.2016**
(45) Mention of the grant of the patent: 17.04.2013
(21) Application number: 07016459.5
(22) Date of filing: 22.08.2007
(51) Int. Cl.: A61K 8/31, A61K 8/41, A61K 8/42, A61K 8/58, A61K 8/891, A61K 8/898, A61Q 5/00, A61Q 5/12

(54) **Aqueous hair cosmetic composition**
Wässrige haarkosmetische Zusammensetzung
Composition cosmétique aqueuse pour les cheveux

(30) Priority: 23.08.2006 JP 2006226042
(43) Date of publication of application: 05.03.2008
(73) Proprietor: Kao Corporation, Chuo-ku Tokyo 103-8210 (JP)
(72) Inventor: Ishino, Yuji, Tokyo 131-8501 (JP)
(74) Representative: Hoffmann Eitle

(56) References cited:
- EP-A- 1 426 027
- WO-A1-01/00140
- WO-A2-01/85108
- JP-A- 5 238 920
- JP-A- 2001 278 732
- JP-A- 2003 012 466
- US-B1- 6 423 303

## Description

### FIELD OF THE INVENTION

The present invention relates to an aqueous hair cosmetic composition containing an alkyl-modified silicone.

### BACKGROUND OF THE INVENTION

When hair is exposed to physical stimulation by daily hair care routines such as heating with a drier, applying a hair iron and brushing, or chemical stimulation by perming, hair coloring or bleaching, it is damaged while suffering a partial loss of the component or structure. Such damage, if it is left untreated, will be a cause of split ends or broken hair, dry and rough to the touch and will create an unmanageable hair style so that it is not preferred to leave it untreated in considering hair care.

To hair cosmetic compositions, various oil components such as silicone oils, ester oils and hydrocarbon oils have conventionally been added by solubilizing, emulsifying or dissolving them in order to provide hair with luster and smoothness without causing a dry and rough feel. In particular, silicone oils have been commonly used in recent years because they have a low surface tension, have a good affinity with hair and provide the hair with good luster.

A hair cosmetic composition using a silicone resin having a specific softening temperature together with a cationic surfactant, a volatile solvent and various modified silicones is disclosed in JP-A-5-238920.

According to this document, the composition can provide the whole hair with a good feel to the touch by efficiently adhering split end portions of the hair. Hair cosmetic compositions containing an alkyl-modified silicone, among various modified silicones, is proposed as a composition excellent in usability upon application and feel to the touch of the hair after drying, and capable of preventing the damage of the hair (JP-A-7-285834 , JP-A-7-285835 and JP-A-2003-12466).

Oil components typified by a silicone oil, however, cannot be used freely because use thereof in a large amount or use thereof by consumers over a long period of time leads to a disadvantage such as increase in friction between individual hairs or greasiness of the hair.

EP-A-1 426 027 relates to compositions comprising crosslinkable siloxane polymers in combination with other polysiloxanes used in cosmetic preparations including hair cosmetic preparations. Example 23 relates to a conditioning mousse comprising dimethyl polysiloxane and alkyl-modified silicones in a weight ratio of the dimethyl polysiloxane to the alkyl-modified silicone of 13.3. Furthermore, stearyldimethylbenzyl ammonium chloride is included in the composition as a cationic surfactant.

US-B1-6.423,303 describes water-in-oil emulsions containing secondary tallow ammonium salts, cyclomethicone and cetyldimethicone copolyol.

JP 2001 278732 A discloses a hair conditioner, comprising: (A) 0.55% stearyl trimethyl ammonium chloride, (B) 0.54% linear dimethyl silicone with the tradename *"KF96A-bcs"* (from Shin-Etsu Chemical), (C) 1.02 % alkyl modified silicone with the formula R₃(R₁)₂Si-O-(SiR₁R₂-O)ₘ - (SiR₁R₁-O)ₙ - SiR₁)₂R₄, wherein R₁=R₃=R₄=CH₃, R₂=CₐH₁₇, m=15, n=15; and water, wherein the ratio (B)/(C)=0.54/1.02=0.5.
WO 01/00140 A1 refers to water-in-oil hair conditioners with lamella dispersion in water phase. A formulation is disclosed which contains (i) 2 wt% Varisoft TA-100 (distearyldimethyl ammonium chloride), (ii) 11 wt% cyclomethicone DC245 (cyclic silicone oil having a boiling point of 260°C or less under ordinary pressure) and (ii) 4 wt% Abil EM 90 (cetyl dimethicone copolyol).

### SUMMARY OF THE INVENTION

In one aspect of the present invention, there is provided an aqueous hair cosmetic composition containing the following components (A), (B), (C) and water:
(A): a cationic surfactant as defined in claim 1
(B): a volatile silicone or volatile hydrocarbon, or mixtures thereof as defined In claim 1
(C): an alkyl-modified silicone as defined in claim 1, at a (B)/(C) weight ratio ranging from 0.1 to 10.

In another aspect of the present invention, there is also provided a treatment method of the hair, which includes applying the above-described aqueous hair cosmetic composition to the hair and then rinsing off the composition.

### DETAILED DESCRIPTION OF THE INVENTION

The present invention relates to an aqueous hair cosmetic composition which has excellent effects for improving the manageability or the styling property of the hair, particularly, does not leave a dry and rough feel of the hair even after drying or one day after application.

The present inventor has found that an aqueous hair cosmetic composition containing an alkyl-modified silicone with a cationic surfactant and a volatile oil as defined in claim 1 can satisfy the above-described demand.

The cationic surfactant as Component (A) includes quaternary ammonium salts represented by the following formula (1): 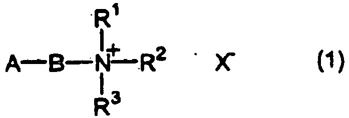 (wherein, A represents a hydrogen atom, or a fatty acid amide, N-substituted carbamoyl, acyloxy or alkoxy group , each having from 12 to 24 carbon atoms in total, B stands for a divalent C₁₋₂₈ hydrocarbon group which may have a hydroxy group, R¹, R² and R³ each represents a C₁₋₃ alkyl group, or at least one of them represents an alkyl or alkenyl group having from 4 to 24 carbon atoms in total and the remainder(s) represents a C₁₋₃ alkyl group, and X⁻ represents a halide ion or an organic anion), and tertiary amine compounds represented by the following formula (2) and salts thereof: 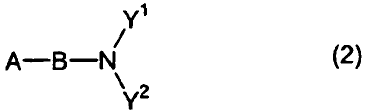 (wherein, A and B have the same meanings as described above and Y¹ and Y² each independently represents a C₁₋₄ alkyl group).

Examples of the quaternary ammonium salt represented by the formula (1) (which will hereinafter be called "quaternary ammonium salt (1)) include mono(long chain) alkyl (C₁₂₋₂₄) quaternary ammonium salts, di (long chain)alkyl or alkenyl (C₁₂₋₂₄) quaternary ammonium salts, branched alkyl (C₁₂₋₂₈) quaternary ammonium salts, fatty acid amido (C₁₂₋₂₄) alkyl (C₁₋₅) quaternary ammonium salts, N-substituted carbamoyl (C₁₂₋₂₄) alkyl (C₁₋₅) quaternary ammonium salts, acyl (C₁₂₋₂₄) oxyalkyl (C₁₋₅) quaternary ammonium salts, and alkyl- or alkenyl (C₁₂₋₂₄) oxyalkyl (C₁₋₅) quaternary ammonium salts.

The mono(long chain)alkyl (C₁₂₋₂₄) quaternary ammonium salts include stearyltrimethylammonium chloride, myristyltrimethylammonium chloride, cetyltrimethylammonium chloride, arachyltrimethylammonium chloride, behenyltrimethylammonium chloride and lauryltrimethylammonium chloride.

The di (long chain) alkyl or alkenyl (C₁₂₋₂₄) quaternary ammonium salts include distearyldimethylammonium chloride, dioleyldimethylammonium chloride, di[(2-dodecanoylamino)ethyl]dimethylammonium chloride and di[(2-stearoylamino)propyl]dimethylammonium ethosulfate.

The branched alkyl (C₁₂₋₂₈) quaternary ammonium salts include diisostearyldimethylammonium methosulfate, 2-decyltetradecyltrimethylammonium chloride, 2-dodecylhexadecyltrimethylammonium chloride, di-2-hexyldecyldimeth-ylammonium chloride and di-2-octyldodecyldimethylammonium chloride.

The fatty acid amido (C₁₂₋₂₄) alkyl (C₁₋₅) quaternary ammonium salts include stearamidopropyl quaternary ammonium salts. The N-substituted carbamoyl (C₁₂₋₂₄) alkyl (C₁₋₅) quaternary ammonium salts include N-stearylcar-bamoylpropyl quaternary ammonium salts. The acyl (C₁₂₋₂₄) oxyalkyl (C₁₋₅) quaternary ammonium salts include stearoxypropyl quaternary ammonium salts. The alkoxy (C₁₂₋₂₄) alkyl (C₁₋₅) quaternary ammonium salts include octadecyloxypropyltrimethylammonium chloride.

In the tertiary amine compound represented by the formula (2) (which will hereinafter be called "tertiary amine compound (2)"), A represents a hydrogen atom, or a fatty acid amide, N-substituted carbamoyl, acyloxy or alkoxy group, each having from 12 to 24 carbon atoms in total, B represents a divalent C₁₋₂₈ hydrocarbon group which may have a hydroxy group, and Y¹ and Y² each independently represents a C₁₋₄ alkyl group.

When A is a group other than a hydrogen atom, it is preferably a fatty acid amide or alkoxy group having from 14 to 22, more preferably from 18 to 22 carbon atoms in total. Further, the hydrocarbon moiety thereof is preferably saturated, more preferably linear and saturated. In this case, B is preferably a trimethylene or 2-hydroxytrimethylene group. When A represents a hydrogen atom, on the other hand, B is preferably a C₁₈₋₂₂ group, more preferably a saturated C₁₈₋₂₂ group, even more preferably a linear saturated C₁₈₋₂₂ group. It has preferably a hydroxy group. Examples of Y¹ and Y² include methyl, ethyl, propyl, isopropyl, butyl and t-butyl groups, of which methyl and ethyl groups are preferred, with a methyl group being more preferred.

As the tertiary amine compound (2), preferred are ether amine compounds and amidoamine compounds represented by the following formulas (2a) and (2b), respectively: 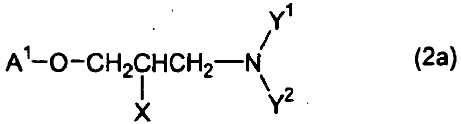 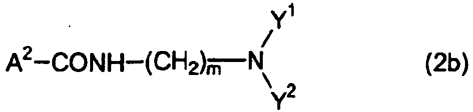 (wherein, Y¹ and Y² have the same meanings as described above, A¹ represents a C₁₂₋₂₄ alkyl or alkenyl group, X represents a hydrogen atom or hydroxy group, A² represents a C₁₁₋₂₃ alkyl or alkenyl group and m stands for a number from 2 to 4).

The ether amine compounds of the formula (2a) adsorb well to the hair and provide the hair with smoothness and softness during application to the hair and rinsing and with a moist feel and smoothness during drying. Specific examples of the ether amine compounds include N,N-dimethyl-3-hexadecyloxypropylamine, N,N-dimethyl-3-octadecyloxypropylamine, N,N-dimethyl-3-hexadecyloxy-2-hydroxypropylamine, N,N-dimethyl-3-octadecyloxy-2-hydroxypropylamine and N,N-dimethyl-3-behenyloxy-2-hydroxypropylamine. It is commercially available, for example, as "Catinal SHPA" (trade name of N,N-dimethyl-3-octadecyloxy-2-hydroxypropylamine; product of Toho Chemical, INCI: Stearyl PG-dimethylamine).

The amidoamine compounds of the formula (2b) adsorb well to the hair and provide the hair with smoothness during rinsing and drying. They also contribute to the dispersion stabilities of other components. Specific examples of the amidoamine compounds include isostearic acid diethylaminoethylamide, oleic acid diethylaminoethylamide, stearic acid diethylaminoethylamide, stearic acid diethylaminopropylamide, stearic acid dibutylaminoethylamide, stearic acid dibutylaminopropylamide, stearic acid dipropylaminopropylamide, stearic acid dipropylaminoethylamide, stearic acid dimethylaminoethylamide, stearic acid dimethylaminopropylamide, palmitic acid diethylaminoethylamide, palmitic acid diethylaminopropylamide, palmitic acid dimethylaminoethylamide, palmitic acid dimethylaminopropylamide, myristic dimethylaminoethylamide, myristic acid dimethylaminopropylamide, behenic acid diethylaminoethylamide, behenicacid diethylaminopropylamide, behenic acid dimethylaminoethylamide, behenic acid dimethylaminopropylamide, arachidic acid dimethylaminoethylamide and arachidic acid dimethylaminopropylamide. Of these, stearic acid diethylaminoethylamide, stearic acid dimethylaminopropylamide and behenic acid dimethylaminopropylamide are more preferred from the standpoints of performance, stability and easy availability.

It is preferred to use the tertiary amine compound (2) as an acid addition salt formed by the addition of at least an equimolar amount of an acid. As the acid used for the formation of the acid addition salt, those used for pH adjustment of the aqueous hair cosmetic composition, which will be described later, are usable. It is possible to allow the acid to serve for both purposes.

The quaternary ammonium salts (1) and tertiary amine compounds (2) as Component (A) may be used either singly or in combination of two or more. The content of Component (A) in the aqueous hair cosmetic composition of the invention is preferably from 0.01 to 20 wt.%, more preferably from 0.05 to 15 wt.%, even more preferably from 0.5 to 10 wt.% in view of providing the hair with smoothness after rinsing or drying. When Component (A) is a salt of the tertiary amine compound, the above-described content means a content in terms of the tertiary amine compound.

Component (B) is represented by low-boiling-point linear silicone oils, low-boiling-point cyclic silicone oils, and low-boiling-point isoparaffin hydrocarbons, each having a boiling point of 260°C or less under ordinary pressure.

The low-boiling-point linear silicone is represented by the following formula: 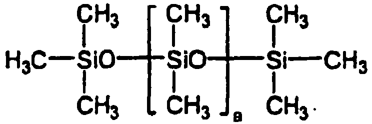 (wherein, a stands for an integer from 0 to 5). Specific examples thereof include hexamethyldisiloxane, octamethyltrlsiloxane, decamethyltetrasiloxane and hexadecamethylheptasiloxane. It is commercially available, for example, as "Silicone KF96A (5cs)" (trade name; product of Shin-Etsu Chemical).

The cyclic silicone is represented by the following formula: 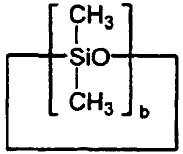 (wherein, b stands for an integer from 3 to 7). Specific examples include octamethylcyclotetrasiloxane, decamethylcyclopentasiloxane, dodeca methylcyclo hexasiloxane and tetradecamethylcycloheptasiloxane. It is commercially available, for example, as "Silicone KF994" or "Silicone KF995", (each, trade name; product of Shin-Etsu Chemical).

Examples of the low-boiling-point isoparaffin hydrocarbon include light Isoparaffin, purified isododecane and mixtures of aliphatic hydrocarbons different in chain length, each having a boiling point ranging from 60 to 260°C under ordinary pressure. It is commercially available, for example, "IP Solvent" (trade name; product of Idemitsu Petrochemical) and "MARUKASOL R" (trade name; product of Maruzen Petrochemical).

Of these, the volatile silicones are preferred, with the cyclic silicones being more preferred. Decamethylcyclopentasiloxane and dodecamethylcyclohexasiloxane are even more preferred because they are relatively stable in an airtight container. Of these volatile silicones, those capable of enhancing drying of the aqueous hair cosmetic composition and providing a non-sticky and fresh feel during use or after completion of the treatment are more preferred.

As Component (B), these volatile silicones or hydrocarbons may be used either singly or In combination of two or more. The total content is preferably from 0.01 to 50 wt. %, more preferably from 0.01 to 20 wt.%, even more preferably from 0.01 to 5 wt.%. The above-described component may be used in combination with a lower alcohol such as ethanol or isopropyl alcohol.

The alkyl-modified silicone as Component (C) is represented by the following formula (3): 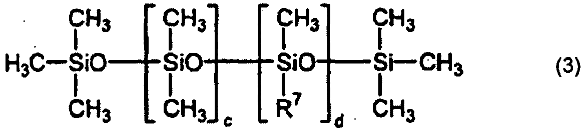 (wherein, R⁴ and R⁵ each independently represents a C₁₋₄ alkyl group, R⁶ represents a C₁₋₄₀ alkyl or alkenyl group, R⁷ represents a C₁₆₋₄₀ alkyl or alkenyl group, c stands for a number of 2 or greater, and d stands for a number of 3 or greater with the proviso that the sum of c and d Is from 200 to 3000).

The alkyl-modified silicone as Component (C) has a melting point from 10 to 60°C, preferably from 20 to 50°C. The melting point of the organopolysiloxane is a value as measured using a differential scanning calorimeter (DSC). The component (C) is represented by the following formula (3): 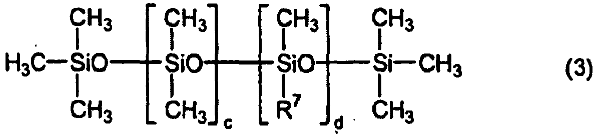 (wherein, c, d and R¹ have the same meanings as described above).

The organosiloxane of the formula (3) is commercially available, for example, as "XF42-A7154". "XF42-A5048". and "XF42-A5047" (each, trade name; product of GE Toshiba Silicone).

These alkyl-modified silicones as Component (C) may be used either singly or In combination of two or more. The content thereof in the aqueous hair cosmetic composition of the present invention is preferably from 0.01 to 20 wt.%, more preferably from 0.01 to 10 wt%, even more preferably from 0.01 to 5 wt.% from the standpoints of improving the manageability or styling property of the hair, suppressing roughness of the hair after drying and making the hair smooth even at the tip of the hair.

In the aqueous hair cosmetic composition of the invention, Component (B) and Component (C) are used so that a (B)/(C) weight ratio falls within a range of from 0.1 to 10 from the standpoints of manageability and styling property of the hair. The ratio within a range of from 0.3 to 3 is preferred in order to improve performances of the composition further.

The aqueous hair cosmetic composition of the present invention may contain silicones other than Components (B) and (C) for the purpose of improving feel to the touch of the hair during rinsing and after drying. Examples of such silicones include dimethylpolysiloxane, amino-modified polysiloxane; fluorine-modified polysiloxane, polyoxyalkylene-modified polysiloxane, carboxylic acid-modified polysiloxane, alcohol-modified polysiloxane and epoxy-modified polysiloxane. Two or more of these silicones may be used in combination. The content thereof in the aqueous hair cosmetic composition of the present invention is preferably from 0.1 to 20 wt%, more preferably from 0.5 to 10 wt.%.

The aqueous hair cosmetic composition of the present Invention may contain an oil component for the purpose of improving feel to the touch (non-greasy feel and moist feel) after drying. Examples of such an oil component include liquid oils or fats such as linseed oil, camellia oil, Macadamia nut oil, corn oil, mink oil, olive oil, avocado oil, sasanqua oil, castor oil, safflower oil, jojoba oil, sunflower oil, almond oil, rapeseed oil, sesame oil, soybean oil, peanut oil, mead-owfoam oil, glyceryl trioctanoate, glyceryl tri-2-ethylhexanoate, and glyceryl triisopalmitate; ester oils such as cetyl octanoate, hexyl laurate, isopropyl myristate, myristyl myristate, isopropyl palmitate, octyl palmitate, hexadecyl palmitate. isocetyl stearate, hydrogenated castor oil stearate, hydrogenated castor oil monohydroxystearate, isopropyl isostearate, octyl isopalmitate, isodecyl oleate, pentaerythritol tetra-2-ethylhexanoate, 2-ethylhexyl succinate and diethyl sebacate; hydrocarbons such as liquid paraffin, squalane, squalene, paraffin and ceresin; and fatty acids such as lauric acid, myristic acid, palmitic acid, oleic acid and stearic acid. Two or more of these oil components may be used in combination. The content thereof in the aqueous hair cosmetic composition of the present invention is preferably from 0.01 to 20 wt. %, more preferably from 0.1 to 10 wit.%.

The aqueous hair cosmetic composition of the present Invention may contain (D) a higher alcohol. Examples of the higher alcohol include cetyl alcohol, cetostearyl alcohol, stearyl alcohol and behenyl alcohol. Of these, linear C₁₈₋₂₄ alkanols are preferred, with stearyl alcohol and behenyl alcohol being more preferred. Two or more of them may be used in combination. The content thereof is preferably from 0.5 to 20 moles, more preferably from 1 to 10 moles, more preferably from 2 to 8 moles, per mole of Component (A) from the standpoints of smoothness during from application to rinsing and even after drying, stability of the system, and adjustment of the viscosity to an adequate level.

The aqueous hair cosmetic composition of the invention may further contain an organic solvent. Examples of the organic solvent include aromatic alcohols such as benzyl alcohol, benzyl oxyethanol and Phenoxyethanol; N-alkylpyr-rolidones such as N-methylpyrrolidone, N-octylpyrrolidone and N-laurylpyrrolidone; alkylene carbonates such as ethylene carbonate and propylene carbonate; and lactones such as γ-butyrolectone and γ-caprolactone. Two or more of these organic solvents may be used in combination. The content thereof in the aqueous hair cosmetic composition of the Invention is preferably from 0.01 to 50 wt.%, more preferably from 0.1 to 35 wt.%, even more preferably from 0.3 to 10 wt.% in order to improve the feeling upon use, luster and flexibility.

The aqueous hair cosmetic composition of the invention may contain a polyol. Examples of the polyol include propylene glycol, 1,3-butanediol, dipropylene glycol, polypropylene glycol (preferably having a molecular weight of from 200 to 700), glycerin, polyglycerins such as diglycerin and triglycerin, trimethylolethane, trimethylolpropane, erythritol, pentaerythritol, sorbitan, glucose, sorbitol, maltitol, sucrose, raffinose, trehalose, polyoxyethylene methyl glucoside, and polyoxypropylene methyl glucoside. Two or more of these polyols may be used in combination. The content thereof in the aqueous hair cosmetic composition of the invention is preferably from 0.01 to 20 wt.%, more preferably from 0.01 to 15 wt.%, even more preferably from 0.01 to 10 wt.% in order to improve the spreadability of the solution at the time of application and smoothness during rinsing.

The aqueous hair cosmetic composition of the present invention may contain a water soluble polymer in order to improve stability of the composition. Examples of the water soluble polymer include cationic polymers, amphoteric polymers, anionic polymers, nonionic polymers and natural polymers and derivatives thereof.

The cationic polymers include cationic cellulose, cationic starch, cationic guar gum, diallyldialkyl quaternary ammonium salt/acrylamide copolymer, and quaternized polyvinylpyrrolidone. Of these, cationic cellulose and cationic guar gum are preferred, with cationic cellulose being more preferred. They are commercially available as "Catinal" series (product of Toho Chemical), and "UCARE POLYMER JR" series and "UCARE POLYMER LR" series (each, product of Amerchol).

The amphoteric polymers include polymers or copolymers of a carboxybetaine monomer such as "PLAS CIZE L401" (trade name; product of GOO Chemical) and "YUKAFORMER AM-75" and "YUKAFORMER AM75S/SM" (each, trade name; product of Mitsubishi Chemical); and acrylic acid/diallyldialkyl quaternary ammonium salt/acrylamide copolymer such as "Merquat Plus 3330" (trade name; product of Nalco) and "AMPHOMER 28-4910" and "AMPHOMER LV-71" (each, trade name; product of National Starch). Of these, "Merquat Plus 3330" and "YUKAFORMER AM-75" are preferred.

Examples of the anionic polymer include acrylate/methacrylate copolymer ("PLAS CIZE", trade name; product of Goo chemical), vinylpyrrolidone/vinyl acetate/vinyl propionate copolymer ("Ruviskol VAP", trade name; product of BASF), vinylpyrrolidone/acrylate copolymer (Luviflex, trade name; product of BASF), and acrylate/acrylamide copolymer ("Ultrahold", product of BASF). Of these, carboxy-containing polymers are preferred.

Examples of the nonionic polymer include polyvinylpyrrolidone such as "Luviskol K12", "Luviskol K17", "Luviskol K30", "Luviskol K60", "Luviskol K80" and "Luviskol K90" (each, trade name; product of BASF), and "PVP K15", "PVP K30", "PVP K60", and "PVP K90 (each, trade name; product of ISP); and "Luviskol VA28E", "Luviskol VA37E", "Luviskol VA55E", "Luviskol VA64E" and "Luviskol VA73E" (each, trade name; product of BASF). Of these, "Luviskol K17", "Luviskol K30" and "PVP K30" are preferred.

Examples of the natural polymer or derivative thereof include natural polysaccharides such as guar gum and xanthan gum; chitosan derivatives such as hydroxypropyl chitosan; starch derivatives such as methylhydroxypropyl starch; cellulose derivatives such as methylhydroxypropyl cellulose, hydroxyethyl cellulose, hydroxypropyl cellulose, carboxymethylcellulose sodium; and alginic acid derivatives such as sodium alginate and propylene glycol alginate. Of these, hydroxypropyl cellulose and hydroxyethyl cellulose are preferred.

One or more of these water soluble polymers are usable. The content thereof in the aqueous hair cosmetic composition of the invention is preferably from 0.01 to 10wt.%, more preferably from 0.05 to 5 wt.%, even more preferably from 0.05 to 2 wt.%.

The pH at 25°C of the aqueous hair cosmetic composition of the invention diluted with water to 20 times its weight is adjusted preferably to from 2 to 6, more preferably from 2.5 to 5, even more preferably from 2.8 to 4.7. Organic acids or inorganic acids are usable for adjusting pH. The organic or inorganic acids similar to those employed for the above-described neutralization of the tertiary amine compounds as Component (A) are usable. Examples of the organic acids include hydroxy acids, monocarboxylic acids, dicarboxylic acids, tricarboxylic acids, polycarboxylic acids, alkylsulfuric acids, and alkylphosphoric acids. The hydroxy acids include glycolic acid, lactic acid, oxybutyric acid, glyceric acid, malic acid and tartaric acid; the monocarboxylic acids include acetic acid; dicarboxylic acids include malonic acid, succinic acid, glutamic acid, adipic acid, maleic acid, fumaric acid, and phthalic acid; and tricarboxylic acids include citric acid. Examples of the inorganic acids include hydrochloric acid, sulfuric acid and phosphoric acid. Of these, the organic acids are preferred, with a -hydroxycarboxylic acids being more preferred. Of these, lactic acid and malic acid are more preferred from the standpoints of luster, flexibility and manageability of the hair. The content of the organic acid or inorganic acid in the aqueous hair cosmetic composition of the invention is preferably from 0.05 to 10 wt.%, more preferably from 0.1 to 5 wt.%.

The aqueous hair cosmetic composition of the invention uses water as a medium. Depending on the purpose of using the composition, it may contain, in addition to the above-described components, surfactants other than Component (A); pH regulators such as sodium hydroxide and potassium hydroxide; chelating agents such as ethylenediaminetetraacetic acid (EDTA); amino acids and derivatives thereof; polymer particles such as polyethylene, polystyrene, polymethylmethacrylate, nylon and silicone, and those having hydrophobic surface treatment; and other additives including humectants, extracts from animals or plants pharmaceutically active ingredients, proteins, antibiotics, ultraviolet absorbers, pearling agents, preservative, bactericides, anti-dandruffs, colorants and perfumes.

The aqueous hair cosmetic composition of the present invention is prepared by mixing Components (A), (B) and (C), other components used as needed depending on the purpose of using the composition, and water. Upon preparation, it is preferred to mix Components (B) and (C) in advance.

The aqueous hair cosmetic composition of the present invention is provided as a hair conditioner, hair treatment, hair pack or the like to be used in a bathroom and washed away after application to the hair.

When the aqueous hair cosmetic composition of the present invention is used as a hair conditioner, it is only necessary to apply it to the hair and then rinse it off with water. This enables to provide the hair with manageability, moist feel, and smoothness after drying without leaving a dry and rough feel on the hair. In addition, combing, brushing or finger combing of the thus-treated hair becomes very smooth.

The following examples further describe and demonstrate embodiments of the present invention. The examples are given solely for the purpose of illustration and are not to be construed as limitations of the present invention.

### -EXAMPLES-

In the following Examples and Comparative Examples, pH is a value as measured at 25°C when each hair cosmetic composition is diluted with water to 20 times its weight.

### Examples 1 to 6 and Comparative Examples 1 and 2

Hair conditioners having the composition shown in Table 1 were prepared and evaluated in the following manners, respectively.

### (Preparation process)

To purified water heated to 80°C were added an acid and a nonionic polymer (hydroxyethyl cellulose) (aqueous phase). Component (A), higher alcohol (stearyl alcohol), oil component and solvent were mixed and dissolved at 80°C (oil phase). The oil phase was added to the aqueous phase while stirring. The mixture was emulsified by stirring for about 30 minutes. The resulting emulsion was cooled to 60°C and a remaining portion of the acid was added thereto, followed by stirring for about 30 minutes. A mixed solution obtained in advance by dissolving Component (C) in Component (B) was added and then, the other silicones were added. After cooling the resulting mixture to 45°C, the remaining components were added thereto and the mixture was cooled to 30°C while stirring.

### (Evaluation method)

Each of the conditioners shown in Table 1 was evaluated in the following manner. About 20 g (about 15 to 20 cm) of the hair of each of twenty Japanese women not exposed to chemical treatments such as permanent waving and dyeing was subjected to bleaching treatment (bath ratio: 1:1) with "Prettia High bleach" (trade name; product of Kao Corp) at 40°C for 20 minutes twice. The hair was then shampooed. The hair conditioner (2 g) was uniformly applied to the hair. After the conditioner was rinsed off with running water for 30 seconds, the hair was blow-dried.

In accordance with the following criteria, the twenty women organoleptically evaluated "manageability", "moist feel", "absence of dry and rough feel", "smoothness", "absence of flyaway hair", "smooth combing, brushing or finger combing" and "conditioning effect on damaged hair". Total scores are shown in Table 1 (maximum score: 100 and minimum score: 20)
5: Very effective
4: Effective
3: Difficult to judge
2: Slightly effective
1: Ineffective

**Table 1**

| Component (wt.%) | | Example | | | | | | Comp.Ex. | |
|---|---|---|---|---|---|---|---|---|---|
| | | 1 | 2 | 3 | 4* | 5 | 6* | 1 | 2 |
| (A) | N,N-Dimethyl-3-octadecyloxypropylamine | | 2.3 | 2.3 | | | 2.3 | | |
| | Stearic acid dimethylaminopropylamide | | | | 1.5 | | | | 1.5 |
| | Behenic acid dimethylaminopropylamide | | | | | 1.5 | | 1.5 | |
| | Stearyltrimethylammonium chloride | 0.1 | | | | | | | |
| | Octadecyloxypropyltrimethylammonium chloride | 2 | | | | | | | |
| (B) | Octamethylcyclotetrasiloxane | | | | 0.2 | | | | 0.2 |
| | Decamethylcyclopentasiloxane | 0.2 | 0.1 | 0.5 | | | | | |
| | Dodecamethylcyclohexasiloxane | | | | | 0.2 | | | |
| | Isoparaffin | | | | | | 0.2 | | |
| | Isododecane | | | | | | 0.1 | | |
| (C) | Octadecyl-modified silicone ¹⁾ Melting point: 40°C | | | | | | 0.1 | | |
| | Octadecyl-modifed silicone ²⁾ Melting point 38°C | | 0.1 | 0.5 | | | | | |
| | Octadecyl-modified silicone ³⁾ Melting point 35°C | | | | 0.3 | | | | |
| | Hexadecyl-modified silicone ⁴⁾ Melting point 27°C | | | | | 0.3 | | 0.3 | |
| | Hexadecyl-modified silicone ⁵⁾ Melting point: 25°C | | | | | | 0.1 | | |
| | Docosyl-modified silicone ⁶⁾ Melting point 55°C | 0.2 | | | | | | | |
| Others | Stearyl alcohol | 8 | 8 | 8 | 8 | 8 | 8 | 8 | 8 |
| | Amino-modified silicone | 0.6 | 0.6 | 0.6 | 0.6 | 0.6 | 0.6 | 0.6 | 0.6 |
| | Methylpolysiloxane (degree of polymerization: 600) | 2.5 | 2.5 | 2.5 | 2.5 | 2.5 | 2.5 | 2.5 | 2.5 |
| | Highly polymerized methylpolysiloxane (average degree of polymerization: 2500) | 1 | 1 | 1 | 1 | 1 | 1 | 1 | 1 |
| | Dipropylene glycol | 3 | 3 | 3 | 3 | 3 | 3 | 3 | 3 |
| | Benzyl alcohol | 0.3 | 0.3 | 0.3 | 0.3 | 0.3 | 0.3 | 0.3 | 0.3 |
| | Hydroxyethyl cellulose | 0.1 | 0.1 | 0.1 | 0.1 | 0.1 | 0.1 | 0.1 | 0.1 |
| | Dicocodimonium chloride | 0.05 | 0.05 | 0.05 | 0.05 | 0.05 | 0.05 | 0.05 | 0.05 |
| | Hybrid sunflower oil | 0.2 | 0.2 | 0.2 | 0.2 | 0.2 | 0.2 | 0.2 | 0.2 |
| | Hydrogenated castor oil monohydroxystearate | 0.3 | 0.3 | 0.3 | 0.3 | 0.3 | 0.3 | 0.3 | 0.3 |
| | pH regulator (lactic acid, glutamic acid, malic acid) | q.s. | q.s. | q.s. | q.s. | q.s. | q.s. | q.s. | q.s. |
| | Perfume | Trace | Trace | Trace | Trace | Trace | Trace | Trace | Trace |
| | Water | Balance | Balance | Balance | Balance | Balance | Balance | Balance | Balance |
| pH (at 25°C, when diluted with water to 20 times the weight) | | 3.3 | 3.3 | 3.3 | 3.3 | 3.3 | 3.3 | 3.3 | 3.3 |
| Evaluation | After drying: manageability of hair | 92 | 92 | 93 | 93 | 91 | 91 | 77 | 61 |
| | After drying: Moist feel of hair | 90 | 92 | 93 | 93 | 92 | 91 | 76 | 69 |
| | After drying: absence of a dry and rough feel | 90 | 92 | 92 | 92 | 91 | 92 | 74 | 66 |
| | After drying: smoothness | 91 | 90 | 92 | 91 | 90 | 89 | 75 | 63 |
| | After drying: absence of flyaway hair | 91 | 93 | 94 | 92 | 91 | 91 | 77 | 61 |
| | After drying: smooth combing, brushing or finger combing | 90 | 90 | 93 | 92 | 90 | 92 | 74 | 65 |
| | After drying: conditioning effect on damaged hair | 93 | 93 | 93 | 93 | 91 | 92 | 72 | 60 |
| 1) formula (3): c=750, d=750, R⁷=C₁₈H₃₇ | | | | | | | | | |
| 2) formula (3): c=500, d=500, R⁷=C₁₈H₃₇ | | | | | | | | | |
| 3) formula (3): c=50, d=50, R⁷=C₁₈H₃₇ | | | | | | | | | |
| 4) formula (3): c=500, d=500, R⁷=C₁₆H₃₃ | | | | | | | | | |
| 5) formula (3): c=75, d=75, R⁷=C₁₆H₃₃ | | | | | | | | | |
| 6) formula (3): c=500, d=500, R⁷C₂₂H₄₅ | | | | | | | | | |
| *: Not falling within the scope of the invention as claimed | | | | | | | | | |

### Example 7: Hair conditioner

| | (wt.%) |
|---|---|
| Behenyltrimethylammonium chloride | 1.5 |
| Stearyl alcohol | 3 |
| Cetyl alcohol | 2 |
| Dipropylene glycol | 5 |
| Glycerin | 1 |
| Docosyl-modified silicone ⁶⁾ | 0.2 |
| Decamethylcyclopentasiloxane | 0.1 |
| Bis-methoxypropylamido isodocosane | 0.2 |
| Dimethicone-containing emulsion | 2.5 |
| ("CF-2460", product of Dow Corning Toray) | |
| Malic acid | 0.1 |
| Lactic acid | 0.7 |
| Glutamic acid | 0.1 |
| Hybrid sunflower oil | 0.5 |
| Hydrolyzed silk | 0.1 |
| Camellia oil | 0.1 |
| Benzyloxyethanol | 0.3 |
| Benzyl alcohol | 0.2 |
| Dipentaerythritol fatty acid ester | 0.2 |
| ("COSMOL 168AR", product of Nisshin Oillio) | |
| Phenoxyethanol | 0.1 |
| Ion exchanged water | Balance |

The above-described hair conditioner (pH 3.2) is excellent in manageability, moist feel, absence of a dry and rough feel, smoothness, and smooth combing, brushing or finger combing of the hair after drying, creates substantially no flyaway hair and is effective for the damaged hair. In addition, it is excellent in stability.

### Example 8: Hair treatment

| | (wt.%) |
|---|---|
| N,N-Dimethyl-3-octadecyloxypropylamine | 2.3 |
| Stearyl alcohol | 8 |
| Dipropylene glycol | 3 |
| Octadecyl-modified silicone ²⁾ | 0.1 |
| Decamethylcyclopentasiloxane | 0.1 |
| Bis-methoxypropylamido isodocosane | 0.5 |
| Amino-modified silicone | 0.6 |
| Highly-polymerized methylpolysiloxane | 1 |
| (average degree of polymerization: 2500) | |
| Dimethylpolysiloxane (degree of polymerization: 600) | 2.5 |
| Malic acid | 0.2 |
| Benzyl alcohol | 0.5 |
| Lactic acid | 1.8 |
| Hybrid sunflower oil | 0.2 |
| Hydrogenated castor oil monohydroxystearate | 0.3 |
| Ion exchanged water | Balance |

The above-described hair treatment (pH 3.3) is excellent in manageability, moist feel, absence of a dry and rough feel, smoothness, and smooth combing, brushing or finger combing after drying, creates substantially no flyaway hair, and is effective for the damaged hair. In addition, it is excellent in stability.

### Example 9: Hair treatment

| | (wt. %) |
|---|---|
| Cetyltrimethylammonium chloride | 0.5 |
| Cetostearyl alcohol | 1.6 |
| Highly polymerized methylpolysiloxane emulsion | 1 |
| ("Silicone BY22-060", product of Dow Corning Toray) | |
| Isopropyl palmitate | 2 |
| Octadecyl-modified silicone ²⁾ | 0.2 |
| Decamethylcyclopentasiloxane | 0.1 |
| Bis-methoxypropylamido isodocosane | 0.3 |
| Amino-modified silicone | 0.05 |
| Malic acid | 0.1 |
| Lactic acid | 0.05 |
| Hybrid sunflower oil | 0.5 |
| Camellia oil | 0.1 |
| Hydrolyzed silk | 0.1 |
| Perfume | 0.03 |
| Ion exchanged water | Balance |

The above-described hair treatment (pH 3.3) is excellent in manageability, moist feel, absence of a dry and rough feel, smoothness, and smooth combing, brushing or finger combing after drying, creates substantially no flyaway hair, and is effective for the damaged hair. In addition, it is excellent in stability.

## Claims

1. An aqueous hair cosmetic composition, comprising the following components (A), (B), (C) and water:
(A): a surfactant, being one or more selected from the group consisting of quaternary ammonium salt compounds represented by the following formula (I): 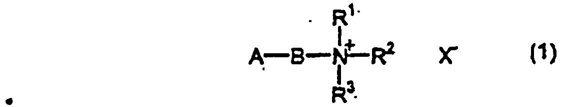 wherein, A represents a hydrogen atom, or a fatty acid amide group, a N-substituted carbamoyl group, an acycloxy group or an alkoxy group, each having from 12 to 24 carbon atoms in total, B represents a divalent C₁₋₂₈ hydrocarbon group which may have a hydroxy group, R¹, R² and R³ each represents a C₁₋₃ alkyl group or at least one of R¹, R² and R³ represent an alkyl or an alkenyl group having from 4 to 24 carbon atoms in total and the remaining group(s) represents a C₁₋₃ alkyl group, and X⁻ represents a halide ion or an organic anion; and tertiary amine compounds represented by the following formula (2) or salts therof: 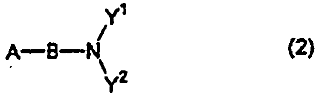 wherein A and B have the same meanings as defined above any Y¹ and Y² each independently represents a C₁₋₄ alkyl group;
(B): a volatile silicone, a volatile hydrocarbon, or mixtures thereof, being selected from linear silicone oils, cyclic silicone oils and isoparaffin hydrocarbons, each having a boiling point of 260°C or less under ordinary pressure;
(C): an alkyl-modified silicone having the following unit (c) and unit (d) in a molecule thereof: 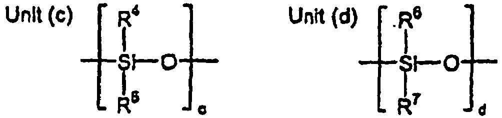 wherein R⁴ and R⁵ each independently represents a C₁₋₄ alkyl group, R6 represents a C₁₋₄₀ alkyl or alkenyl group, R⁷ represents a C₁₆₋₄₀ alkyl or alkenyl group, c stands for a number of 2 or greater, and d stands for a number of 3 or greater with the proviso that the sum of c and d is from 200 to 3000;
wherein the (B)/(C) weight ratio ranges from 0.1 to 10; wherein the melting point of the alkyl-modified silicone is from 10°C to 60°C as measured using a differential scanning calorimeter (DSC); and
wherein the component (C) is represented by the following formula (3): 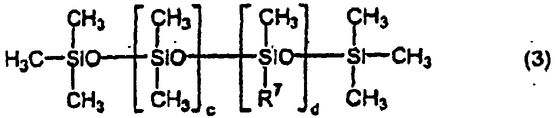 wherein R⁷ represents a C₁₆₋₄₀ alkyl or alkenyl group, c stands for a number of 2 or greater and d stands for a number of 3 or greater with the proviso that the sum of c and d is from 200 to 3000.

2. The aqueous hair cosmetic composition according to Claim 1, wherein Component (B) is a cyclic silicone.

3. The aqueous hair cosmetic composition according to Claim 1, further comprising a linear C₁₈₋₂₄ alkanol as Component (D).

4. The aqueous hair cosmetic composition according to Claim 1, which has a pH from 2 to 6 at 25°C when diluted with water to 20 times the weight.

5. A hair treatment method, which comprises applying the aqueous hair cosmetic composition according to any one of Claims 1 to 4 to the hair and then rinsing off the composition.

## Patentansprüche

1. Wässrige Haarkosmetikzusammensetzung, die die folgenden Komponenten (A), (B), (C) und Wasser umfasst:
(A) ein Tensid, das eines oder mehrere ausgewählt aus der Gruppe ist, bestehend aus quaternären Ammoniumsalzverbindungen der folgenden Formel (1): 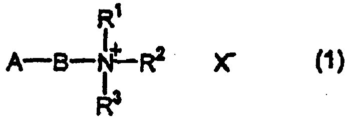 worin A ein Wasserstoffatom oder eine Fettsäureamidgruppe, eine N-substituierte Carbamoylgruppe, eine Acyloxygruppe oder eine Alkoxygruppe, die jeweils insgesamt von 12 bis 24 Kohlenstoffatome aufweisen, darstellt, B eine zweiwertige C₁₋₂₈-Kohlenwasserstoffgruppe darstellt, die eine Hydroxygruppe aufweisen kann, R¹, R² und R³ jeweils eine C₁₋₃-Alkylgruppe darstellen oder mindestens eines aus R¹, R² und R³ eine Alkyl- oder eine Alkenylgruppe mit von 4 bis 24 Kohlenstoffatomen insgesamt darstellt und die verbleibenden Gruppen eine C₁₋₃-Alkylgruppe darstellen, und X⁻ ein Halogenidion oder ein organisches Anion darstellt; und tertiären Aminverbindungen der folgenden Formel (2) oder Salzen davon: 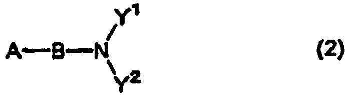 worin A und B die gleiche Bedeutung wie oben definiert und Y¹ und Y² jeweils unabhängig voneinander eine C₁₋₄-Alkylgruppe darstellen;
(B) ein flüchtiges Silikon, ein flüchtiger Kohlenwasserstoff oder Mischungen davon, die aus der Gruppe bestehend aus linearen Silikonölen, cyclischen Silikonölen und Isoparaffin-Kohlenwasserstoffen ausgewählt sind, die jeweils einen Siedepunkt von 260°C oder weniger unter Normaldruck aufweist;
(C) ein Alkyl-modifiziertes Silikon, das die folgende Einheit (c) und die Einheit (d) in einem Molekül davon aufweist: 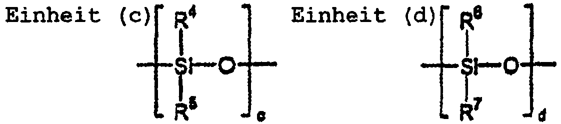 worin R⁴ und R⁵ jeweils unabhängig voneinander eine C₁₋₄-Alkylgruppe darstellen, R⁶ eine C₁₋₄₀-Alkyl- oder -Alkenylgruppe darstellt, R⁷ eine C₁₆₋₄₀-Alkylgruppe- oder -Alkenylgruppe darstellt, c für eine Zahl von 2 oder größer steht und d für eine Zahl von 3 oder größer steht, mit der Bedingung, dass die Summe von c und d von 200 bis 3.000 ist;
worin das (B)/(C)-Gewichtsverhältnis im Bereich von 0,1 bis 10 liegt;
worin der Schmelzpunkt des Alkyl-modifizierten Silikons von 10 bis 60°C ist, gemessen unter Verwendung eines Differentialrasterkalorimeters (DSC); und
worin Komponente (C) durch die folgende Formel (3) dargestellt wird: 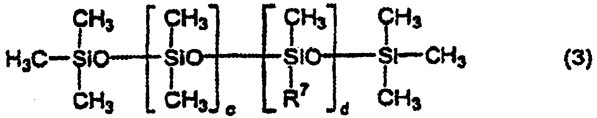 worin R⁷ eine C₁₆₋₄₀-Alkylgruppe oder -Alkenylgruppe darstellt, c für eine Zahl von 2 oder größer steht und d für eine Zahl von 3 oder größer steht mit der Bedingung, dass die Summe von c und d von 200 bis 3.000 ist.

2. Wässrige Haarkosmetikzusammensetzung gemäß Anspruch 1, worin Komponente (B) ein cyclisches Silikon ist.

3. Wässrige Haarkosmetikzusammensetzung gemäß Anspruch 1, die weiterhin ein lineares C₁₈₋₂₄-Alkanol als Komponente (D) umfasst.

4. Wässrige Haarkosmetikzusammensetzung gemäß Anspruch 1, die einen pH-Wert von 2 bis 6 bei 25°C aufweist, wenn sie mit Wasser auf das 20-fache des Gewichts verdünnt wurde.

5. Haarbehandlungsverfahren, das die Anwendung der wässrigen Haarkosmetikzusammensetzung gemäß irgendeinem der Ansprüche 1 bis 4 auf das Haar und dann das Abwaschen der Zusammensetzung umfasst.

## Revendications

1. Composition cosmétique aqueuse pour les cheveux comprenant les composants (A), (B), (C) suivants et de l'eau :
(A) : un agent tensioactif, étant un ou plusieurs choisi(s) parmi le groupe consistant en des composés de sel ammonium quaternaire représentés par la formule (1) suivants : 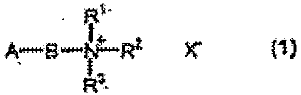 dans laquelle, A représente un atome d'hydrogène, ou un groupe amide d'acide gras, un groupe carbamoyle N-substitué, un groupe acyloxy ou un groupe alcoxy, chacun ayant de 12 à 24 atomes de carbone au total, B représente un groupe hydrocarbure en C₁-C₂₈ divalent qui peut avoir un groupe hydroxy, R¹, R² et R³ représentent chacun un groupe alkyle en C₁-C₃ ou bien au moins un parmi R¹, R² et R³ représente un groupe alkyle ou alcényle ayant de 4 à 24 atomes de carbone au total et le/les groupe(s) restant(s) représente (nt) un groupe alkyle en C₁-C₃, et X⁻ représente un ion halogénure ou un anion organique ; et des composés d'amine tertiaire représentés par la formule (2) suivante ou des sels de ceux-ci : 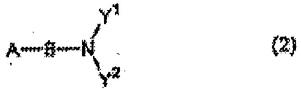 dans laquelle A et B ont les mêmes sens que définis ci-dessus et Y¹ et Y² représentent chacun indépendamment un groupe alkyle en C₁-C₄ ;
(B) : une silicone volatile, un hydrocarbure volatile, ou des mélanges de ceux-ci, étant choisi parmi des huiles de silicone linéaire, des huiles de silicone cyclique et des hydrocarbures isoparaffiniques, chacun ayant un point d'ébullition de 260 °C ou moins à la pression ordinaire ;
(C) : une silicone modifiée avec un alkyle ayant le motif (c) et le motif (d) suivants dans une molécule de celle-ci : 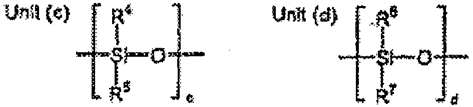 dans lesquels R⁴ et R⁵ représentent chacun indépendamment un groupe alkyle en C₁-C₄, R⁶ représente un groupe alkyle ou alcényle en C₁-C₄₀, R⁷ représente un groupe alkyle ou alcényle en C₁₆-C₄₀. c représente un nombre de 2 ou plus, et d représente un nombre de 3 ou plus sous réserve que la somme de c et d soit de 200 à 3 000 ;
dans laquelle le rapport en poids (B)/(C) est dans la plage de 0,1 à 10 ;
dans laquelle le point de fusion de la silicone modifiée avec un alkyle est de 10 °C à 60 °C tel que mesuré au moyen d'un calorimètre différentiel à balayage (DSC) ; et
dans laquelle le composant (C) est représenté par la formule (3) suivante : 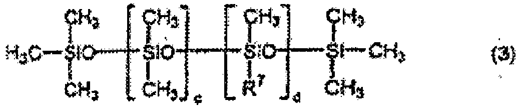 dans laquelle R⁷ représente un groupe alkyle ou alcényle en C₁₆-C₄₀, c représente un nombre de 2 ou plus, et d représente un nombre de 3 ou plus sous réserve que la somme de c et d soit de 200 à 3 000.

2. Composition cosmétique aqueuse pour les cheveux selon la revendication 1, dans laquelle le Composant (B) est une silicone cyclique.

3. Composition cosmétique aqueuse pour les cheveux selon la revendication 1, comprenant en outre un alcanol linéaire en C₁₈-C₂₄ comme Composant (D).

4. Composition cosmétique aqueuse pour les cheveux selon la revendication 1, qui a un pH de 2 à 6 à 25 °C lorsqu'elle est diluée avec de l'eau à hauteur de 20 fois le poids.

5. Procédé de traitement des cheveux, qui comprend l'application de la composition cosmétique aqueuse pour les cheveux selon l'une quelconque des revendications 1 à 4 aux cheveux et ensuite le rinçage de la composition.
